# EUROPEAN PATENT APPLICATION

(11) **EP 2 067 491 A1**
(43) Date of publication of application: **10.06.2009**
(21) Application number: 08020822.6
(22) Date of filing: 01.12.2008
(51) Int. Cl.: A61L 9/03

(54) **USB-powered air-freshener**

(30) Priority: 04.12.2007 US 992149 P
(71) Applicant: Givaudan SA, 1214 Vernier (CH)
(72) Inventor: McGee, Thomas, Cottesloe, WA 6011 (AU); Litten-Brown, Colin, RG42 2BD Bracknell (GB)
(74) Representative: Givaudan Patents

(57) **Abstract**

A volatile liquid emitting device, comprising an electrical heating element and a reservoir of volatile liquid, liquid and heating element being disposed in a heating relationship to each other and the heating element receiving electricity from an integral USB connector, the volatilised liquid passing into the atmosphere through a membrane that is impermeable to the liquid but permeable to the vapour thereof.

## Description

This invention relates to air fresheners, and more particularly to air fresheners adapted to be heated by means of a universal series bus (USB) connection.

USB connectors are now universally fitted on computers, plus many other types of electronic apparatus, such as game systems, and they serve to power a wide variety of computer-related peripheral equipment. It has been proposed to have a USB-operated device for the release into the atmosphere of a volatile liquid, typically a fragrance for use in air freshening. Such a device would have the capacity to enhance substantially a working environment, by providing a pleasant odour and counteracting the staleness often found in such working environments.

Such devices have been made, but none has been completely satisfactory. One commercial device involves pouring a liquid into a cavity that is provided with a USB-powered heating element. Such a device brings the dangers of spillage or leakage, with possible damage to the computer itself. The use in the cavity of an absorbent pad only partially alleviates this potential problem. Another device seeks to avoid this by using a fragranced rod, which is inserted into the device. This is limiting insofar as heating may not be optimal. An additional problem is that is it not possible to provide any end-of-life indication.

It has now been found that it is possible to provide fragrance in the vicinity of a device having a USB connection, such that there is efficient fragrance release and no danger of leakage, all in a compact unit. There is therefore provided a volatile liquid emitting device, comprising an electrical heating element and a reservoir of volatile liquid, liquid and heating element being disposed in a heating relationship to each other and the heating element receiving electricity from an integral USB connector, the volatilised liquid passing into the atmosphere through a membrane that is impermeable to the liquid but permeable to the vapour thereof.

There is additionally provided a method of providing a volatile liquid in the atmosphere surrounding an apparatus comprising a USB connection, comprising the volatilisation of liquid from a reservoir thereof by heating by means of an electrical heating element that derives its electrical current from the USB connection, the evaporated liquid entering the atmosphere through a membrane covering the reservoir, which is impermeable to the liquid but permeable to the evaporated liquid.

Basically, the device is a small unit with an integral USB connector and a reservoir of volatile liquid.

The reservoir that holds the volatile liquid may be an integral part of the device, but in a particular embodiment, it is a removable refill, which fits into a matching chamber in the device, allowing easy refill and change of liquid, should this be desired. The reservoir, be it integral or removable, may be made in any desired shape or configuration. Given the nature of the device, it will naturally be relatively small, typically holding a few ml of liquid. It may also be transparent or translucent, such that the quantity of liquid therein may be observed, thus giving an end-of-life indication.

In a particular embodiment, the reservoir is a two-chamber reservoir, that is, the reservoir has the form of two chambers, in liquid communication with other by means of a relatively narrow passage joining them. In one particular embodiment, one of the reservoir chambers has a volume considerably smaller than that of the other chamber, the smaller chamber only being heated. This embodiment is further discussed hereinunder.

There are known to the art many liquid-impermeable but vapour-permeable membranes, and any such suitable membrane may be used. The membrane will naturally be expected to be chemically resistant to the liquid for the duration of its service life. The membrane may also advantageously be transparent, to allow visual end-of-life determination. Typical examples of suitable membranes include, but are not limited to polyethylene, polypropylene, polystyrene, polyvinyl chloride, polyvinyl acetate, polyamide, polyacrylamide, polymethacrylate, co-extrusion of polypropylene and low density polyethylene and the like, with a thickness of 15 to 150 micrometers.

Prior to use, the membrane surface is covered with a closure element, such as a cover, seal, lid or other sealing means. This may be, for example, a polymeric film or a metal foil, typically of aluminium, or a laminate of one or more layers of, for example, low density polyethylene, aluminum foil, and polyester. Attachment to the container may be achieved by means of, for example, an adhesive, a heat-sealable lacquer or a heat-sealable polymer, these having sufficient adhesive strength to prevent premature opening, but permitting the closure element to be peeled away from the container by hand when the container is to be opened. The membrane may also be part of a frangible laminate, one part of the laminate being peelable.

Membranes and closure elements as hereinabove described are readily available commercially, for example, from the US company Rollprint Packaging Products, Inc., of Addison, IL.

The heating element may be any suitable heating element that conveys sufficient heat to the liquid to cause it to vaporise and pass through the membrane in sufficient quantity to have the desired beneficial effect on the atmosphere. The heating element may be one that is located within the liquid in the reservoir, or which is in a heating relationship adjacent to the reservoir. In the latter case, it may be built into the structure of the reservoir, or, in the case of a removable reservoir, the reservoir may be seated on the element or on a surface comprising the element. In the case of an element acting as a seat, the surface on which the reservoir rests may be flat, or it may be any other suitable or desired shape. In one embodiment, the surface has, in cross-section, a sawtooth configuration, with that part of the reservoir in contact therewith having a matching configuration. This not only provides a larger surface for heat transfer, but also allows the heating elements themselves to be placed in the "valleys" between the sawtooth "peaks", thus preventing their being touched by accident.

The selection of the type and configuration of the heating element is well within the skill of the art and such selection requires only routine experimentation. The heater element should naturally be such that it generates sufficient heat to vaporise the liquid, but not so much that it could cause physical damage to the device or the computer or constitute a hazard of any kind. In the case of a heating element within a liquid, it should ideally be positioned on or near the bottom of the reservoir.

The heating element may be a simple wire with suitable resistance characteristics to allow the necessary heat generation. This wire may be straight or coiled, in the manner of an incandescent light bulb. The heating element may have ancillary elements, for protection or for better heat transfer, or both. For example, the wire may be attached by any suitable means to the underside of a flat plate of suitable configuration, on which the reservoir rests. Alternatively, the heating element may be shrouded in a protective covering of a heat-resistant material, such as ceramic or silica.

The temperature attained at the conducting surface/heating element interface should be sufficient for the purpose. It will be limited to some extent by the electricity supply from a USB connector and by the nature of the liquid itself, but in general the surface temperature of the element should not exceed 70°C.

The electricity for the heating element is supplied by a USB connector integral with the device. By "integral" is meant that the reservoir, the heating element and the connector form a single compact unit, with no cables involved. The physical size of the unit will depend to some extent on the quantity of liquid required, but in general it will comprise a unit about the same size as on of the larger memory sticks or mini-drives in common use for storing information. The USB connector is an entirely standard type.

The heat supplied by the heating element should be sufficient such that a desired quantity of liquid be emitted into the atmosphere. The achievement of this is well within the skill of the art, but some factors that play a part in it are
- the nature of the liquid/heating interface.
- the thickness of the membrane.

As has already been seen, the heating element may be in direct contact with the liquid, or it may be remote from, but in heat transfer contact with, it. In the latter case, the heating element may be built into the sides or bottom of the reservoir, or, in the case of a removable and replaceable reservoir, it may be in a surface against which the reservoir rests. One of the surprising features of this device is that even a reservoir made of plastic with a low thermal conductivity can conduct sufficient heat from a low wattage USB circuit to provide sufficient liquid to the atmosphere, provided the membrane is of a suitable thickness and porosity. While a suitable membrane is easily provided, as a general guide, a membrane of thickness of less than 150 micrometres is desirable.

In a further embodiment, that part of the reservoir in contact with or adjacent to the heating element may have enhanced thermal conductivity. There are a number of ways of doing this. For example, the contact surface may contain metal or metal oxide powder, to enhance heat transfer. The powder may be any suitable powder, for example, iron filings, copper or aluminium powder or powdered iron oxide. Alternatively, the surface may be a laminated material, of, for example, polymeric material and metal.

In a further embodiment, that part of the reservoir in contact with or adjacent to the heating element may be made of metal sheet or foil. Examples include iron, aluminium and copper. Iron is useful because it may be magnetised, which may be useful in holding a removable reservoir in place. In a particular embodiment, the contact surface has a conductivity measured at 25 C of at least 100W. m⁻¹.K⁻¹, more particularly 200W. m⁻¹.K⁻¹. Aluminium and copper have conductivities of 230W.m⁻¹.K⁻¹ and 410W.m⁻¹.K⁻¹, respectively.

In a further particular embodiment, the reservoir has the form of an elongate tray with a surface of enhanced thermal conductivity forming a base and the membrane a top cover. The sides of the tray may be formed of any suitable material. For example, it may be formed from the same material as the surface of enhanced conductivity, or from a different material. In a particular embodiment, it may be polymeric. The side material should be such that it can be adhered to both conductive surface and membrane to form a liquid-tight enclosure. Typical side materials include, but are not limited to, polyethylenes, polypropylenes, polyvinyl chlorides, polyphenolsulfide, ethylene-vinyl acetate copolymers, ethylene-acrylic acid ester copolymers and the like.

In a further particular embodiment, as previously described, the reservoir is formed as a two-chamber reservoir, that is, the reservoir has the form of two chambers, in liquid communication with other by means of a relatively narrow passage joining them, this passage extending all the way to the floors of the chambers. In one particular variation of this embodiment, one of the reservoir chambers has a volume considerably smaller than that of the other chamber, and the heat transfer contact between reservoir and heating element is only with the smaller chamber. This has the advantage of minimising the heating of the case. In such an embodiment, if it is desired to enhance the thermal conductivity of the reservoir material, only that of the chamber to be heated need be enhanced.

In a particular embodiment, the device will comprise a chamber with a USB connector built into one end thereof, the chamber being adapted to hold a removable and replaceable reservoir, the chamber having a heating element that will heat the conductive surface of a reservoir placed therein.

The device is easy and inexpensive to produce, using readily-available materials and techniques. It will be appreciated that, if desired, other features may be added to the device to enhance its utility. These may include on-off switches, light-emitting diodes to indicate proper functioning, and intensity controls, such as variable resistors or slidable covers.

The device is convenient to use and is effective in use. Moreover, it is spill-proof and with an appropriate design gives an indication of end-of-life. In the embodiment that comprises reservoirs as refills, refilling is easily accomplished and a range of different fragrances can easily be made available.

The device will now be further described with reference to the accompanying drawings, which depict preferred embodiments and which are in no way intended to be limiting on the scope of this disclosure.

Figure 1 shoes a perspective view of a device.

Figure 2 is a transverse vertical cross-section of the device of Figure 1.

Figure 3 is a longitudinal vertical cross-section of the device of Figure 1.

Figure 4 is a perspective view from below of a particular reservoir according to the invention.

Figure 5 is a schematic plan view of the reservoir of Figure 4, showing part of the construction in detail.

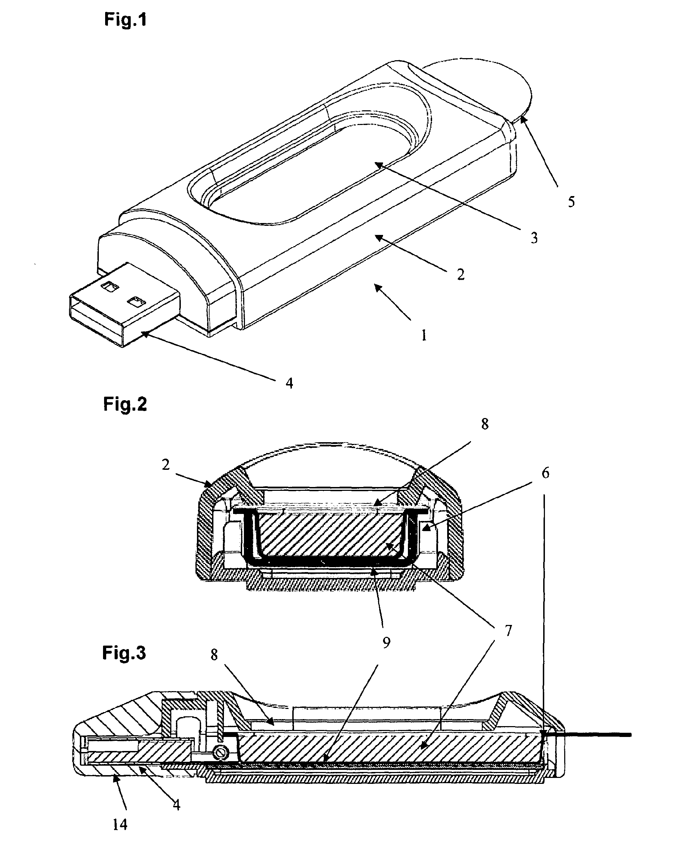

In Figures 1-3, a device generally indicated as 1 comprises a body portion 2 with an opening 3 at the top. The device is equipped with an integral USB connector 4, which may be plugged into any apparatus with a suitable connector, such as a computer or a video game apparatus. Prior to use, the USB connector is protected by a cover 14 (Figure 3). Within the body proportion is located a removable reservoir, which at one end comprises a tab 5 for easy removal. The body portion has a snap-fit cover, which can be removed for the removal and replacement of the reservoir.

The reservoir comprises a tray 6 containing volatile liquid 7 for evaporation into the atmosphere. The tray is vacuum-formed from APET (amorphous polyethylene terephthalate) and having a top portion of polyethylene copolymer capable of being heat-bonded to a membrane and a protective layer (further described hereinunder). The APET is loaded with aluminium powder to facilitate heat conductivity.

The upwardly-facing opening of the tray is covered with a porous membrane 8, chosen such that it is impermeable to the liquid but permeable to the vapour. The membrane is a 35VX (88 micron) polyolefin membrane from Rollprint Packaging Products, Inc., Addison, IL USA

The reservoir is seated on a heating element 9, which draws electrical power from the USB connector. The heating element is an aluminium tray, on which the reservoir sits, and to the underside of which is attached an insulated resistance wire.

The reservoir is supplied with a peelable vapour-tight protective, which is removed before use. The material used is a breakaway laminate, comprising an aluminium foil bonded to a polymer laminate, and heat bonded to the reservoir (the material used is RPP# 26-1219 (0.002" oPET/ 0.0005"LDPE/0.001" aluminium/ break-away layer/ 0.0015" membrane) of Rollprint Packaging Products, Inc., Addison, IL USA).

In use, the protective foil is removed, the reservoir is inserted into the body portion and the device plugged into an apparatus by means of the USB connector 4. Electric current from the apparatus enters the device via the USB connector and passes through the heating element 9, causing it to heat. This heat passes through the material of the reservoir 6 to the liquid 7, causing it to evaporate. The evaporated liquid passes through the porous membrane 8 into the atmosphere.

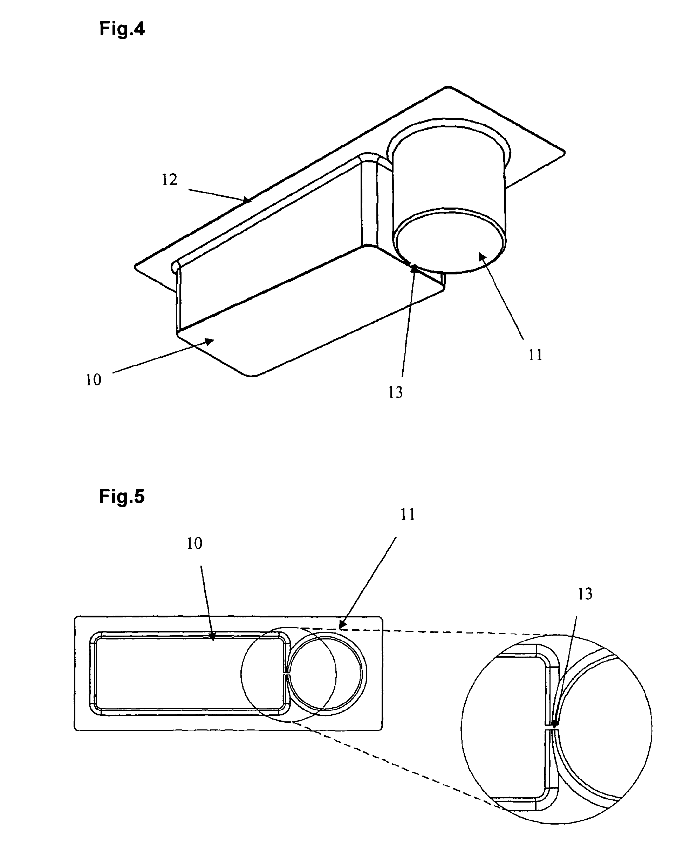

Figures 4 and 5 depict another kind of reservoir. In this case, the reservoir has the form of two reservoirs, 10 and 11, depending from a common top plate 12. Reservoir 10 is rectangular in plan view and is substantially larger in volume than the circular reservoir 11. The two approach each other closely and are joined at the point of maximum closeness by a narrow channel 12, which extends from the top plate to the bottoms of the reservoirs, thus affording a passage through which liquid can flow from one to the other.

The device for use with such a reservoir may be identical to that shown in Figures 1-3, with the difference that the heating element is arranged such that only reservoir 11 is heated, reservoir 10 acting as a liquid supply to replenish the liquid of reservoir 11 as it evaporates. This arrangement permits a much lower temperature within the device.

The skilled person will appreciate that there are many other possible embodiments that lie within the scope of this invention, all of which are readily realisable by the application of the ordinary skill of the art.

## Claims

1. A volatile liquid emitting device, comprising an electrical heating element and a reservoir of volatile liquid, liquid and heating element being disposed in a heating relationship to each other and the heating element receiving electricity from an integral USB connector, the volatilised liquid passing into the atmosphere through a membrane that is impermeable to the liquid but permeable to the vapour thereof.

2. A device according to claim 1, in which the liquid is held in a removable and replaceable container.

3. A device ac cording to claim 1, in which the reservoir is a two-chamber reservoir, having the form of two chambers, in liquid communication with other by means of a relatively narrow passage joining them.

4. A device according to claim 3, in which one of the reservoir chambers has a smaller volume than the other, the smaller chamber only being heated.

5. A device according to claim 2, in which the container rests on the heating element.

6. A device according to claim 5, in which the surface of the heating element has a sawtooth form in cross-section, that part of the reservoir in contact therewith having a matching configuration.

7. A device according to claim 1, in which that part of the reservoir in contact with or adjacent to the heating element is made of metal sheet or foil.

8. A method of providing a volatile liquid in the atmosphere surrounding an apparatus comprising a USB connection, comprising the volatilisation of liquid from a reservoir thereof by heating by means of an electrical heating element that derives its electrical current from the USB connection, the evaporated liquid entering the atmosphere through a membrane covering the reservoir, which is impermeable to the liquid but permeable to the evaporated liquid.
